# EUROPÄISCHE PATENTSCHRIFT

(11) **EP 0 837 046 B1**
(45) Veröffentlichungstag und Bekanntmachung des Hinweises auf die Patenterteilung: **17.10.2001**
(21) Anmeldenummer: 97117243.2
(22) Anmeldetag: 06.10.1997
(51) Int. Cl.: C07C 17/363, C07C 25/13

(54) **Verfahren zur Herstellung von o-Alkylfluorbenzolen**
Process for preparing o-alkyl-fluoro-benzenes
Procédé pour la préparation d'alkyle benzènes orthofluorés

(30) Priorität: 17.10.1996 DE 19642868
(43) Veröffentlichungstag der Anmeldung: 22.04.1998
(73) Patentinhaber: BAYER AG, 51368 Leverkusen (DE)
(72) Erfinder: Lui, Norbert, Dr., 51061 Köln (DE); Rock, Michael-Harold, Dr., 51065 Köln (DE)

(56) Entgegenhaltungen:
- DE-A- 4 225 763

## Beschreibung

Die vorliegende Erfindung betrifft ein Flüssigphasen-Verfahren zur Herstellung von gegebenenfalls substituierten o-Alkylfluorbenzolen aus substituierten Chlorameisensäurephenylestern.

Zur Herstellung von o-Alkylfluorbenzolen, welche als Wirkstoffzwischenprodukte zunehmdem Interesse unterliegen, gibt es bisher nur wenig vorteilhafte Verfahren. Die Blockierung unerwünschter Substitutionsstellen durch tertiäre Butylgruppen erlaubt die selektive Einführung von Fluor an aromatischen Kernen durch Nitrieren, Reduzieren, Verkochen in Gegenwart von Fluoridionen (Baltz-Schiemann-Reaktion) und Abspalten der Butylgruppen (J. Chem. Soc. Perkin Trans. I **1987,** 1).

Aus der DE 42 25 763 A1 ist bekannt, daß man einige Fluorbenzole aus den entsprechenden Chlorameisensäureestern durch Erhitzen in Gegenwart von Fluorwasserstoff und in Abwesenheit von Lösungsmitteln in Ausbeuten von maximal 69 % herstellen kann. Beim Einsatz des Lösungsmittels Trichlortrifluorethan wurde eine deutliche Verschlechterung der Raum-Zeit-Ausbeute und des Umsatzes, auch bei Temperaturerhöhung, gefunden. 2,6-Dimethylfluorbenzol konnte so nur in einer Ausbeute von 40 % erhalten werden.

Es wurde nun ein Verfahren zur Herstellung von o-Alkylfluorbenzolen der Formel (I) 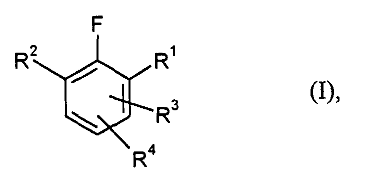 in welcher
- R¹: für C₁-C₆-Alkyl oder C₃-C₆-Cycloalkyl steht,
- R²: für Wasserstoff, C₁-C₆-Alkyl oder C₃-C₆-Cycloalkyl steht,
- R³ und R⁴: unabhängig voneinander für Wasserstoff, Halogen, C₁-C₆-Alkyl, C₃-C₆-Cycloalkyl, C₁-C₆-Alkoxy, Difluormethoxy, Trifluormethoxy, Chlordifluormethoxy, C₁-C₆-Alkylthio, Trifluormethylthio, Di-(C₁-C₄-alkyl)-amino oder Phenyl stehen,
gefunden,
das dadurch gekennzeichnet ist, daß man Chlorameisensäureester der Formel (II) 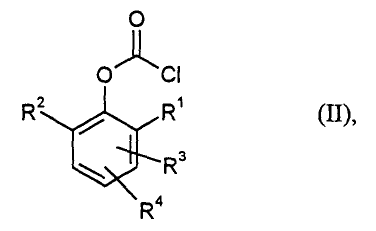 in welcher
- R¹ bis R⁴: die bei Formel (I) angegebenen Bedeutungen haben,
in Gegenwart von Fluorwasserstoff und in Gegenwart eines inerten Verdünnungsmittels in flüssiger Phase auf 70 bis 200°C erhitzt, wobei halogenierte Alkane als inerte Verdünnungsmittel ausgenommen sind.

Vorzugsweise stehen in den Formeln (I) und (II)
- R¹: für geradkettiges oder verzweigtes C₁-C₄-Alkyl oder C₅-C₆-Cycloalkyl,
- R²: für Wasserstoff, geradkettiges oder verzweigtes C₁-C₄-Alkyl oder C₅-C₆-Cycloalkyl und
- R³ und R⁴: unabhängig voneinander für Wasserstoff, Fluor, Chlor, Brom, geradkettiges oder verzweigtes C₁-C₄-Alkyl C₅-C₆-Cycloalkyl, geradkettiges oder verzweigtes C₁-C₄-Alkoxy, Difluormethoxy, Trifluormethoxy, Chlordifluormethoxy, geradkettiges oder verzweigtes C₁-C₄-Alkylthio, Trifluormethylthio, durch gleiches oder verschiedenes geradkettiges oder verzweigtes C₁-C₄-Alkyl disubstituiertes Amino oder für Phenyl.

Besonders bevorzugt stehen
- R¹: für Methyl, Ethyl, n-Propyl oder Isopropyl,
- R²: für Wasserstoff, Methyl, Ethyl, n-Propyl oder Isopropyl und
- R³ und R⁴: unabhängig voneinander für Wasserstoff, Fluor, Chlor, Brom, Methyl, Ethyl, n-Propyl oder Isopropyl, Methoxy, Difluormethoxy, Trifluormethoxy, Chlordifluormethoxy, Methylthio oder für Phenyl.

Die zur Durchführung des erfindungsgemäßen Verfahrens als Ausgangsverbindungen benötigten Chlorameisensäureester der Formel (II) sind bekannt oder können analog zu bekannten Verbindungen hergestellt werden.

Der für die Reaktion benötigte Fluorwasserstoff sollte möglichst wasserfrei sein. Es kommt insbesondere der handelsübliche wasserfreie Fluorwasserstoff (100%-ige Flußsäure) in Frage. Pro Mol Chlorameisensäureester der Formel (II) können z.B. 1 bis 200 Mol, vorzugsweise 1 bis 100 Mol, besonders bevorzugt 2 bis 50 Mol Fluorwasserstoff eingesetzt werden.

Für die Durchführung des erfindungsgemäßen Verfahrens bevorzugte inerte Verdünnungsmittel sind inerte organische Lösungsmittel mit Ausnahme halogenierter Alkane, wobei reine Lösungsmittel oder Gemische davon eingesetzt werden können. Dabei kann die Mischung mit Fluorwasserstoff ein Mehrphasensystem sein. Besonders bevorzugte Verdünnungsmittel sind mehrfach chlorierte Benzole, beispielsweise Dichlorbenzole, insbesondere o-Dichlorbenzol oder das technische Dichlorbenzol-Isomerengemisch, Trichlorbenzole, insbesondere 1,2,3-Trichlorbenzol oder 1,2,4-Trichlorbenzol oder Tetrachlorbenzole, insbesondere 1,2,4,5-Tetrachlorbenzol.

Die zur Durchführung des Verfahrens eingesetzte Menge Verdünnungsmittel kann in großem Maße variiert werden. Im allgemeinen kann man pro Mol Chlorameisensäureester 100 bis 5000 ml, vorzugsweise 150 bis 1000 ml Verdünnungsmittel verwenden.

Das Verfahren kann z.B. durchgeführt werden, indem man Verdünnungsmittel, Fluorwasserstoff und Chlorameisensäureester vorlegt und auf die Reaktionstemperatur aufheizt. Besonders vorteilhaft ist es, den Fluorwasserstoff und ein Teil oder die gesamte Menge des Verdünnungsmittels bei Reaktionstemperatur vorzulegen und den Chlorameisensäureester, der gegebenenfalls in dem anderen Teil des Verdünnungsmittels gelöst sein kann, zuzudosieren. In beiden Fällen läßt man vorzugsweise den entstehende Chlorwasserstoff und das Kohlendioxid über eine Druckhaltung aus dem Reaktionsgefäß entweichen.

Bevorzugte Reaktionstemperaturen liegen zwischen 70 und 180°C. Hinsichtlich der Reaktionstemperatur ist zu beachten, daß diese mindestens so hoch gewählt wird wie die Temperatur, bei der das jeweilige Edukt beginnt zu decarboxylieren. Diese Mindesttemperatur kann gegebenenfalls durch routinemäßige Vorversuche leicht ermittelt werden.

Während der Durchführung des erfindungsgemäßen Verfahrens muß der Druck mindestens so hoch sein, daß sich das Edukt und das Lösungsmittel bei der jeweiligen Reaktionstemperatur ganz überwiegend in flüssiger Phase befinden. Nach oben hin ist der Druck nicht kritisch. Er kann z.B. 0,5 bis 10 x 10⁶ Pa betragen.

Es ist überraschend, daß die Ausbeuten durch die erfindungsgemäße Verwendung von Verdünnungsmitteln erheblich gegenüber dem Stand der Technik gesteigert werden können.

Die Umalkylierung, die insbesondere bei der Decarboxylierung von Trimethylphenylchlorameisensäureestern in HF nach bisherigen Verfahren auftritt, wird beim erfindungsgemäßen Verfahren überaschenderweise zurückgedrängt.

### Beispiele

### Beispiel 1

### 1-Fluor-2,4,6-trimethylbenzol

In einem 2 1-Autoklaven aus Edelstahl wurden unter Rühren 500 g 2,4,6-Trimethylphenylchlorameisensäureester, 600 ml 1,2,4-Trichlorbenzol und 400 ml HF auf 110°C erhitzt. Während 6-stündigem Rühren bei dieser Temperatur wurden die entstehenden Gase (Chlorwasserstoff und Kohlendioxid) über einen solegekühlten Kondensator mit Druckhalteventil (2,5 x 10⁶ Pa) entspannt. Anschließend wurde HF im Vakuum entfernt und der Rückstand auf Wasser gegossen. Die organische Phase wurde abgetrennt und über Natriumsulfat getrocknet. Das Produkt wurde durch Destillation gereinigt. Das Lösungsmittel kann wieder verwendet werden.
Ausbeute: 251 g (72 % d. Th.)

### Beispiel 2

### 1-Fluor-2,3-dimethylbenzol

Es wurde analog Beispiel 1 gearbeitet, jedoch 220 g 2,3-Dimethylphenylchlorameisensäureester, 500 ml 1,2,4-Trichlorbenzol und 600 ml HF auf 140°C erhitzt und bei 2,8 x 10⁶ Pa entstehende Gase entspannt.
Ausbeute: 113 g (76 % d. Th.)

### Beispiel 3

### 1-Fluor-2,6-dimethylbenzol

Es wurde analog Beispiel 1 gearbeitet, jedoch 400 g 2,4-Dimethylphenylchlorameisensäureester, 700 ml Trichlorbenzol und 400 ml HF auf 150°C erhitzt.
Ausbeute: 192 g (71 % d. Th.)

### Beispiel 4

### 1-Fluor-2,6-dimethylbenzol

Es wurde analog Beispiel 1 gearbeitet, jedoch 276 g 2,6-Dimethylphenylchlorameisensäureester, 500 ml 1,2,4-Trichlorbenzol und 600 ml HF auf 140°C erhitzt.
Ausbeute: 172 g (92 % d. Th.)

## Patentansprüche

1. Verfahren zur Herstellung von o-Alkylfluorbenzolen der Formel (I) 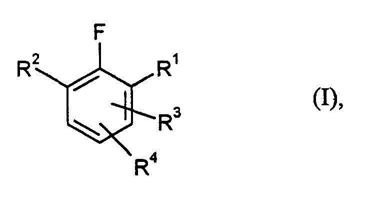 in welcher
R¹ für C₁-C₆-Alkyl oder C₃-C₆-Cycloalkyl steht,
R² für Wasserstoff, C₁-C₆-Alkyl oder C₃-C₆-Cycloalkyl steht,
R³ und R⁴ unabhängig voneinander für Wasserstoff, Halogen, C₁-C₆-Alkyl, C₃-C₆-Cycloalkyl, C₁-C₆-Alkoxy, Difluormethoxy, Trifluormethoxy, Chlordifluormethoxy, C₁-C₆-Alkylthio, Trifluormethylthio, Di-(C₁-C₄-alkyl)-amino oder Phenyl stehen,
gefunden,
**dadurch gekennzeichnet, daß** man Chlorameisensäureester der Formel (II) 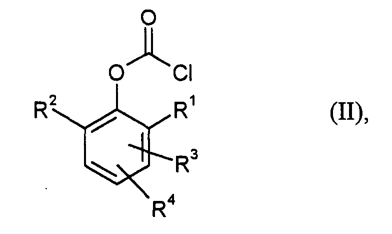 in welcher
R¹ bis R⁴ die bei Formel (I) angegebenen Bedeutungen haben,
in Gegenwart von Fluorwasserstoff und in Gegenwart eines inerten Verdünnungsmittels in flüssiger Phase auf 70 bis 200°C erhitzt, wobei halogenierte Alkane als inerte Verdünnungsmittel ausgenommen sind.

2. Verfahren nach Anspruch 1, **dadurch gekennzeichnet, daß** in den Formeln (I) und (II)
R¹ für geradkettiges oder verzweigtes C₁-C₄-Alkyl oder C₅-C₆-Cycloalkyl,
R² für Wasserstoff, geradkettiges oder verzweigtes C₁-C₄-Alkyl oder C₅-C₆-Cycloalkyl und
R³ und R⁴ unabhängig voneinander für Wasserstoff, Fluor, Chlor, Brom, geradkettiges oder verzweigtes C₁-C₄-Alkyl, C₅-C₆-Cycloalkyl, geradkettiges oder verzweigtes C₁-C₄-Alkoxy, Difluormethoxy, Trifluormethoxy, Chlordifluormethoxy, geradkettiges oder verzweigtes C₁-C₄-Alkylthio, Trifluormethylthio, durch gleiches oder verschiedenes geradkettiges oder verzweigtes C₁-C₄-Alkyl disubstituiertes Amino oder für Phenyl, stehen.

3. Verfahren nach Ansprüchen 1 und 2, **dadurch gekennzeichnet, daß** in den Formeln (I) und (II)
R¹ für Methyl, Ethyl, n-Propyl oder Isopropyl,
R² für Wasserstoff, Methyl, Ethyl, n-Propyl oder Isopropyl und
R³ und R⁴ unabhängig voneinander für Wasserstoff, Fluor, Chlor, Brom, Methyl, Ethyl, n-Propyl oder Isopropyl, Methoxy, Difluormethoxy, Trifluormethoxy, Chlordifluormethoxy, Methylthio oder für Phenyl, stehen.

4. Verfahren nach Ansprüchen 1 bis 3, **dadurch gekennzeichnet, daß** man pro Mol Chlorameisensäureester der Formel (II) 1 bis 200 mol Fluorwasserstoff einsetzt.

5. Verfahren nach Ansprüchen 1 bis 4, **dadurch gekennzeichnet, daß** man als Verdünnungsmittel mehrfach chlorierte Benzole einsetzt.

6. Verfahren nach Ansprüchen 1 bis 5, **dadurch gekennzeichnet, daß** man pro Mol Chlorameisensäureester der Formel (II) 100 bis 500 ml Verdünnungsmittel einsetzt.

7. Verfahren nach Ansprüchen 1 bis 6, **dadurch gekennzeichnet, daß** man es bei 70 bis 180°C durchführt.

8. Verfahren nach Ansprüchen 1 bis 7, **dadurch gekennzeichnet, daß** der Druck mindestens so hoch ist, daß sich das Edukt und das Lösungsmittel bei der jeweiligen Reaktionstemperatur ganz überwiegend in flüssiger Phase befinden.

9. Verfahren nach Ansprüchen 1 bis 8, **dadurch gekennzeichnet, daß** der Druck im Bereich 0,5 bis 10 x 10⁶ Pa liegt.

## Claims

1. Process for preparing o-alkylfluorobenzenes of the formula (I) 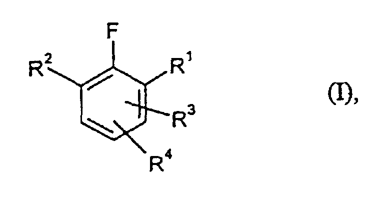 in which
R¹ is C₁-C₆-alkyl or C₃-C₆-cycloalkyl,
R² is hydrogen, C₁-C₆-alkyl or C₃-C₆-cycloalkyl,
R³ and R⁴ independently of one another are each hydrogen, halogen, C₁-C₆-alkyl, C₃-C₆-cycloalkyl, C₁-C₆-alkoxy, difluoromethoxy, trifluoromethoxy, chlorodifluoromethoxy, C₁-C₆-alkylthio, trifluoromethylthio, di-(C₁-C₄-alkyl)amino or phenyl,
**characterized in that** chloroformates of the formula (II) 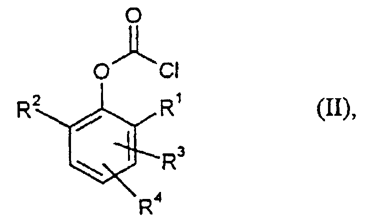 in which
R¹ to R⁴ are each as defined in formula (I),
are heated to from 70 to 200°C in the liquid phase in the presence of hydrogen fluoride and in the presence of an inert diluent other than a halogenated alkane.

2. Process according to Claim 1, **characterized in that** in the formulae (I) and (II)
R¹ is straight-chain or branched C₁-C₄-alkyl or C₅-C₆-cycloalkyl,
R² is hydrogen, straight-chain or branched C₁-C₄-alkyl or C₅-C₆-cycloalkyl and
R³ and R⁴ independently of one another are each hydrogen, fluorine, chlorine, bromine, straight-chain or branched C₁-C₄-alkyl, C₅-C₆-cycloalkyl, straight-chain or branched C₁-C₄-alkoxy, difluoromethoxy, trifluoromethoxy, chlorodifluoromethoxy, straight-chain or branched C₁-C₄-alkylthio, trifluoromethylthio, amino which is disubstituted by identical or different straight-chain or branched C₁-C₄-alkyl, or phenyl.

3. Process according to Claims 1 and 2, **characterized in that** in the formulae (I) and (II)
R¹ is methyl, ethyl, n-propyl or isopropyl,
R² is hydrogen, methyl, ethyl, n-propyl or isoproyl and
R³ and R⁴ independently of one another are each hydrogen, fluorine, chlorine, bromine, methyl, ethyl, n-propyl or isopropyl, methoxy, difluoromethoxy, trifluoromethoxy, chlorodifluoromethoxy, methylthio or phenyl.

4. Process according to Claims 1 to 3, **characterized in that** 1 to 200 mol of hydrogen fluoride are employed per mole of chloroformate of the formula (II).

5. Process according to Claims 1 to 4, **characterized in that** polychlorinated benzenes are used as diluents.

6. The process according to Claims 1 to 5, **characterized in that** 100 to 500 ml of diluent are employed per mole of chloroformate of the formula (II).

7. Process according to Claims 1 to 6, **characterized in that** Process is carried out at 70 to 180°C.

8. Process according to Claims 1 to 7, **characterized in that** the pressure is at least high enough for the reactant and the solvent to be very predominantly in the liquid phase at the respective reaction temperature.

9. Process according to Claims 1 to 8, **characterized in that** the pressure is in the range from 0.5 to 10 x 10⁶ Pa.

## Revendications

1. Procédé pour la préparation d'o-alkylfluorobenzènes de la formule (I) 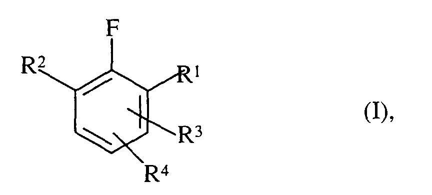 dans laquelle
R¹ représente un groupe alkyle en C₁-C₆ ou cycloalkyle en C₃-C₆,
R² représente un atome d'hydrogène, un groupe alkyle en C₁-C₆ ou cycloalkyle en C₃-C₆,
R³ et R⁴ représentent indépendamment un atome d'hydrogène, d'halogène, un groupe alkyle en C₁-C₆, cycloalkyle en C₃-C₆, alcoxy en C₁-C₆, difluorométhoxy, trifluorométhoxy, chlorodifluorométhoxy, alkylthio en C₁-C₆, trifluorométhylthio, di-(alkyle en C₁-C₄)-amino ou phényle,
**caractérisé en ce que** l'on chauffe un ester d'acide chloroformique de la formule (II) 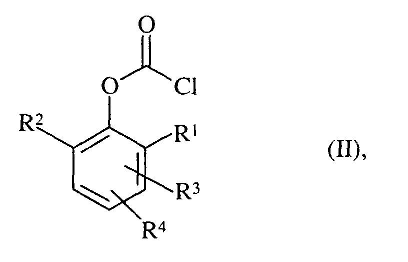 dans laquelle
R¹ à R⁴ ont les significations indiquées dans la formule (I),
en présence d'acide fluorhydrique et en présence d'un agent diluant inerte en phase liquide à de 70 à 200°C, les alcanes halogénés étant exclus en tant qu'agents diluants inertes.

2. Procédé selon la revendication 1, **caractérisé en ce que** dans les formules (I) et (II)
R¹ représente un groupe alkyle en C₁-C₄ linéaire ou ramifié ou cycloalkyle en C₅-C₆,
R² représente un atome d'hydrogène, un groupe alkyle en C₁-C₄ linéaire ou ramifié ou cycloalkyle en C₅-C₆ et
R³ et R⁴ représentent indépendamment un atome d'hydrogène, de fluor, de chlore, de brome, un groupe alkyle en C₁-C₄ linéaire ou ramifié, cycloalkyle en C₅-C₆, alcoxy en C₁-C₄ linéaire ou ramifié, difluorométhoxy, trifluorométhoxy, chlorodifluorométhoxy, alkylthio en C₁-C₄ linéaire ou ramifié, trifluorométhylthio, amino disubstitué par l'intermédiaire de groupes alkyle en C₁-C₄ linéaires ou ramifiés, identiques ou différents ou un groupe phényle.

3. Procédé selon les revendications 1 et 2, **caractérisé en ce que** dans les formules (I) et (II)
R¹ représente le groupe méthyle, éthyle, n-propyle ou isopropyle,
R² représente un atome d'hydrogène, le groupe méthyle, éthyle, n-propyle ou isopropyle et
R³ et R⁴ représentent indépendamment un atome d'hydrogène, de fluor, de chlore, de brome, le groupe méthyle, éthyle, n-propyle ou isopropyle, méthoxy, difluorométhoxy, trifluorométhoxy, chlorodifluorométhoxy, méthylthio ou phényle.

4. Procédé selon les revendications 1 à 3, **caractérisé en ce que** l'on utilise par mole d'ester d'acide chloroformique de la formule (II) de 1 à 200 mol d'acide fluorhydrique.

5. Procédé selon les revendications 1 à 4, **caractérisé en ce que** l'on utilise comme agent diluant des benzènes plusieurs fois chlorés.

6. Procédé selon les revendications 1 à 5, **caractérisé en ce que** l'on utilise par mole d'ester d'acide chloroformique de la formule (II) de 100 à 500 ml d'agent diluant.

7. Procédé selon les revendications 1 à 6, **caractérisé en ce que** l'on réalise la réaction à de 70 à 180°C.

8. Procédé selon les revendications 1 à 7, **caractérisé en ce que** la pression est au moins celle à laquelle l'éduit et le solvant se trouvent de manière complètement prédominante en phase liquide à la température correspondante de la réaction.

9. Procédé selon les revendications 1 à 8, **caractérisé en ce que** la pression se trouve dans le domaine de 0,5 à 10 x 10⁶ Pa.
